(19) Europäisches Patentamt / European Patent Office / Office européen des brevets

(11) **EP 4 200 600 B1**

(12) **EUROPEAN PATENT SPECIFICATION**

(45) Date of publication and mention
of the grant of the patent:
**25.06.2025 Bulletin 2025/26**

(21) Application number: **21859109.7**

(22) Date of filing: **19.08.2021**

(51) International Patent Classification (IPC):
*G01N 22/00* (2006.01)   *G01N 33/18* (2006.01)

(52) Cooperative Patent Classification (CPC):
**G01N 22/00; G01N 33/1813**

(86) International application number:
**PCT/US2021/046623**

(87) International publication number:
**WO 2022/040385 (24.02.2022 Gazette 2022/08)**

(54) **CONTINUOUS HEAVY METAL WATER CONTAMINANT MEASUREMENT SYSTEM**

SYSTEM ZUR KONTINUIERLICHEN MESSUNG VON
SCHWERMETALL-WASSERVERUNREINIGUNGEN

SYSTÈME DE MESURE CONTINU DE CONTAMINANT D'EAU PAR MÉTAL LOURD

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.08.2020   US 202063067691 P**
**04.09.2020   US 202063074730 P**

(43) Date of publication of application:
**28.06.2023 Bulletin 2023/26**

(73) Proprietor: **Marquette University**
**Milwaukee, WI 53201-1881 (US)**

(72) Inventors:
• **LEE, Chung, Hoon**
**Milwaukee, WI 53201-1881 (US)**
• **RICHIE, James**
**Milwaukee, WI 53201-1881 (US)**
• **MEDEIROS, Henry, Ponti**
**Milwaukee, WI 53201-1881 (US)**

(74) Representative: **Slingsby Partners LLP**
**1 Kingsway**
**London WC2B 6AN (GB)**

(56) References cited:
**US-A- 4 480 239       US-A- 4 651 085**
**US-A- 5 103 180       US-A- 5 194 815**
**US-A1- 2015 103 859       US-A1- 2016 334 343**

• **ALBISHI ALI M., RAMAHI OMAR M.: "Highly Sensitive Microwaves Sensors for Fluid Concentration Measurements", IEEE MICROWAVE AND WIRELESS COMPONENTS LETTERS, IEEE SERVICE CENTER, NEW YORK, NY., US, vol. 28, no. 4, 1 April 2018 (2018-04-01), US**
**, pages 287 - 289, XP055908220, ISSN: 1531-1309, DOI: 10.1109/LMWC.2018.2805866**

**Description**

CROSS-REFERENCE TO RELATED APPLICATIONS

The present application claims priority of U.S. Provisional Patent Application No. 63/067,691, filed on August 19, 2020. The present application also claims priority of U.S. Provisional Patent Application No. 63/074,730, filed on September 4, 2020.

BACKGROUND

Current technologies for heavy metal detection include inductive coupled plasma mass spectrometry (ICP-MS), fluorescence spectroscopy, atomic absorption spectroscopy, electrochemical analysis, etc. Detection methods can vary depending on the measurement of real-time parameters.

US5103180 discloses a resonator that has a body with a planar surface, an aperture through the body and a gap extending from the planar surface to the aperture.

BRIEF DISCLOSURE

The present invention provides a resonator including a body, the body having a planar surface. An aperture through the body is configured to receive a tube configured for a fluid to be tested. A gap extends into the body from the planar surface to the aperture. At least one cut extends through the body from the planar surface towards the aperture. The at least one cut extends across the gap.

In examples, the body is cuboid. The at least one cut may be perpendicular to the gap. The at least one cut extends from the planar surface to the aperture. The at least one cut extends from the planar surface through the aperture. The resonator may include at least two cuts. The resonator is configured to produce a resonant frequency that corresponds to a water exchange rate of an ion to be detected by the resonator. The resonator may further include a dielectric material in the gap. The resonator exhibits a resonant frequency for each gap or cut in the resonator.

A system for the detection of an ion in a fluid includes a resonator as described above. A sample tube extends through the aperture of the resonator, the sample tube is configured to receive a sample of the fluid with the ion. A coupling loop includes a coil of wire. The coil of wire of the coupling loop is coaxially aligned with the aperture of the resonator. The sample tube further extends through the coupling loop. A vector network analyzer (VNA) is connected to the coupling loop. The VNA supplies an RF energy signal to the coupling loop. The RF energy signal is transferred to the resonator by inductive coupling. The VNA receives a reflected portion of the RF energy signal through the coupling loop and calculates a reflection coefficient. A processor receives the reflection coefficient and produces a determination if the ion is in the fluid based upon the reflection coefficient.

In examples of the system, the processor applies a support vector regressor (SVR) model to the reflection coefficient to produce the determination if the ion is in the fluid. The processor further determines a concentration of the ion in the fluid by applying the SVR model to the reflection coefficient. The SVR model is produced by a machine learning algorithm trained on datasets of reflection coefficient measurements of fluids having known ion concentrations. The SVR model is specific to a target ion and the SR model is produced by the machine learning algorithm trained on datasets of reflection coefficient measurements of the fluid having known concentrations of the target ion. The resonator is configured to produce a resonant frequency that corresponds to a water exchange rate of the target ion. The resonator is configured to produce a resonant frequency that corresponds to a water exchange rate of the target ion. In an example the target ion is lead and the resonant frequency is 7GHz.

The present invention also provides a method of detecting an ion in a fluid performed with any of the resonators or systems as described above. Examples of the method further include providing the resonator about the tube configured to receive a fluid to be tested. A magnetic field is generated with the resonator. Reflection coefficients are measured as a function of frequency across a frequency range. The measured reflection coefficients are analyzed. A detection of the ion in the fluid is determined based upon the measured reflection coefficients.

Examples of the method include a frequency range between 10 MHz - 10 GHz. Other examples of the method include a frequency range between 10MHz - 5 GHz. The measured reflection coefficients are analyzed using an ion-specific model. The measured reflection coefficients are analyzed using a model produced by training a machine learning algorithm. The model is a support vector regressor model trained using the reflection coefficients measured from a plurality of samples of known ion concentration. The support vector regressor model is trained using the reflection coefficients within a passband centered on a frequency that corresponds to a water exchange rate of the target ion. Analyzing the measured reflection coefficients includes analyzing the measured reflection coefficients within a passband centered on a frequency that corresponds to a water exchange rate of the target ion. In an example, the target ion is lead and the frequency is 7GHz. The resonator is configured to produce a resonant frequency that corresponds to a water exchange rate of a target ion to be detected in the fluid.

BRIEF DESCRIPTION OF THE DRAWINGS

Figure 1A is a perspective view of an example of a block loop gap resonator.
Figure 1B is an end view of an example of a block loop gap resonator.
Figure 2A and 2B are further examples of block loop gap resonators.
Figure 3 is a system diagram of a system for ion detection or concentration measurement.
Figure 4 is a graph of example water exchange rate constants.

DETAILED DISCLOSURE

Sensing systems and methods are desired to meet the sensitivity, selectivity, sensor lifetime, and real-time measurement requirements for water contamination monitoring, particularly for heavy metal detection.

A continuous, static, and non-interfering water contaminant detection system and method is disclosed herein in which RF microwave principles are used to measure specific contaminants found in water. In one example ($NaCl$, $MgCl2$, and a mixture of $NaCl$ and $MgCl2$) are the representative contaminants used in reducing the detection system and method to practice. Another example described herein is the detection of lead (Pb) in a municipal water system. Other examples will be described herein and recognized from the present disclosure.

A loop gap resonator is a microwave device consisting of a hollow cylindrical loop. The hollow cylindrical loop has a consistent wall thickness. The hollow cylindrical loop includes a gap through the wall which extends for the length of the hollow cylindrical loop. Such a device is also known as a slotted tube cavity or a split ring resonator. The device can be considered as a lumped LC circuit where the loop acts as an inductor and the gap is a capacitor. In the presence of an electromagnetic signal, a magnetic field is introduced around the loop. The gap holds electric charges that create a uniform electric field. When the stored magnetic energy in the loop and stored electric energy in the gap are equal, the loop gap resonator becomes resonant.

As will be described herein, microwave measurements and machine learning algorithms are used to detect metal ions, exemplarily in drinking water. As disclosed in further detail herein, a block loop gap resonator (BLGR) is used as a sensor in a system for detecting or quantifying ion concentrations in an analyzed fluid. The BLGR is sensitive to different ions and their concentration resulting in an RF reflection spectrum (S11) amplitude and phase variations in the 10MHz to 10GHz range, while in other examples, narrower or larger frequency ranges may be used. A support vector regressor (SVR) algorithm is used, for example, to classify among detection of different ions and to measure a concentration of a heavy metal (e.g. Pb) ion in water. As disclosed herein, BLGRs may further exhibit multiple resonances, which have been found to enhance the sensitivities of these detections.

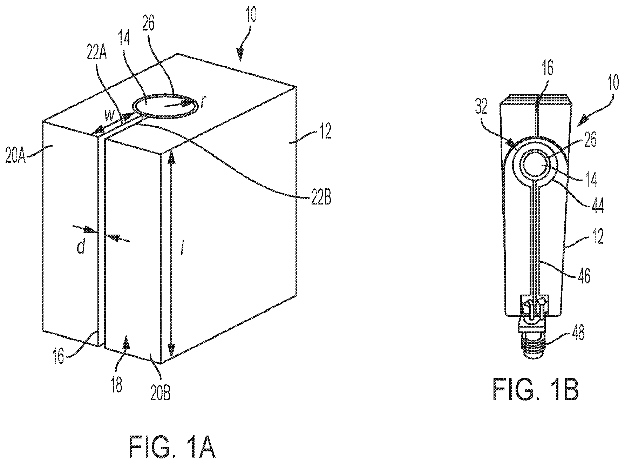

Figures 1A and 1B provide examples of a block loop gap resonator (BLGR) 10 that is not within the scope of the present invention. The BLGR 10 includes a resonator body 12 of a material with a known conductivity (e.g. Aluminum). The resonator body is exemplarily a cuboid block of material with the disclosed features machined therein. It will be recognized that further examples of the BLGR 10 may be formed by casting rather than machining. An aperture 14 with a radius (r) extends through the length (1) of the resonator body 12. The aperture 14 defines the loop of an inductor as will be described herein. A gap 16 extends from an outer surface 18 of the resonator body, through a portion of the body 12, to the aperture 14. The gap 16 separates the resonator body 12 into two conductors 20A, 20B The conductors 20A, 20B each include an opposing surface 22A, 22B that face each other across the gap 16. The gap 16 has a gap depth (d) which is the space between the opposing surfaces 22A, 22B. The width (w) of the gap 16 is the shortest distance from aperture 14 to the outer surface at the gap 16.

With reference to Fig. 1B, a coupling loop 32 is secured to the resonator body 12. The coupling loop 32 includes a coil of wire 44, a transmission line 46, and a connector 48. In examples, the connector 48 may be an SMA (SubMiniature version A) coaxial RF connector. In further examples, the coupling loop 32 may be arranged or oriented in an alternative manner such as to reduce the length of the transmission line 46. In certain examples, reduced transmission line length may result in reduced sensor noise.

A glass tube 26 extends through the aperture 14. A portion of the glass tube 26 is thus surrounded by the resonator body 12 and by the coupling loop 32. A sample to be analyzed is held within the glass tube 26. The sample may be a static sample, e.g. if the ends of the tube are stopped, or the sample may be a continuous flow through the glass tube 26. In examples herein, the sample is water, which may be municipal water, that contains one or more metal ions. As will be described in further detail herein, an external magnetic field generated by the coupling loop 32 is used to continuously measure changes in radio frequency energy in response to the magnetic field. The non-contact feature of the device allows a long sensor lifetime with high sensitivity for real-time measurements.

The output signal of the coupling loop 32 is characterized as the reflection coefficient (S11), as will be described in further detail herein. However, this output signal exhibits many peaks in the signal as a function of frequency. In examples, the output signal may exhibit 10, 20, 25, or more peaks across an operational frequency of 10MHz to 10GHz. These peaks are due to sensor design and the substance being tested. However, both the target ions for analysis, as well as the background chemistry of the substance being tested contribute to the peaks in the output signal. The complexity of the output signal and multiple sources contributing to the output signal present challenges in the simultaneous monitoring and analysis of the frequency and amplitude changes of all of these peaks continuously and in real-time. Therefore, multiple features of the presently disclosed BLGR system improve and facilitate this analysis.

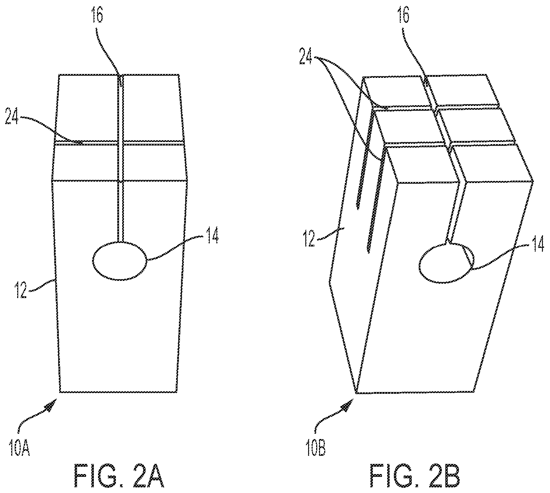

It has been discovered that the BLGR 10 has increased sensitivity to frequencies within a band surrounding a resonant frequency of the sensor. The BLGR 10 depicted in Fig. 1A has a single resonant frequency attributable to the gap 16. However, multi-resonance BLGRs are disclosed herein. Figures 2A and 2B depict examples of multi-resonance BLGR devices 10A, 10B, which are in accordance with the present invention. The multi-resonance BLGR is formed with one or more cuts 24 through the resonator body 12 across the gap 16. In examples as shown in Figs. 1A and 1B, a BLGR 10 with a gap 16, but without cuts 24 has a single resonance. A single cut 24 perpendicular to the gap 16 results in a second resonance, while a second cut 24 (Fig. 2B) parallel to the first cut 24 and perpendicular to the gap 16 results in a third resonance. A single perpendicular cut 24 splits the total capacitance in two, resulting in two resonances in the same structure. While the cuts 24 reduce Q, as will be explained in further detail below, the cuts 24 increase the frequency range across which the BLGR is most sensitive to measurements of particular ions for detection. The BLGR can be designed to have multiple resonant frequencies by introducing cuts to the structure. While Fig. 2A depicts one cut 24 and Fig. 2B depicts two cuts 24, respectively, it will be recognized that in other various BLGR designs, more cuts may be introduced, including but not limited to, up to six or more cuts.

It will be recognized that the at least one cut 24 may extend into the resonator body 12 from the outer surface 18 of the body 12 across the gap 16 to the aperture 14. In examples, the gap 16 is aligned with the axis of the aperture 14 through the body 12, and therefore the gap 16 connects to the aperture 14 at the smallest distance from the outer surface 18 to the aperture 14, for a width (w). However, since the cuts 24 are exemplarily perpendicular to the gap 16, the cuts 24 may extend into the body 12 at various depths relative to the aperture 14. In an example the cuts 24 extend into the body 12 the same distance (w) as the gap 16. In other examples, the cuts 24 extend through the aperture 14. The cuts 24 may extend to the furthest point of the aperture 14 from the outer surface, or the cuts 24 may terminate at a distance within the aperture 14. As noted above, Figs. 2A and 2B respectively depict BLGR 10A and 10B which both include at least one cut 24 through the gap 16 from the outer surface 18 through the resonator body 12 to the aperture 14. The at least one cut 24 may be perpendicular to the gap 16. In an example, the cut(s) 24 may be in a plane perpendicular to the plane of the gap 16. In still further examples, the at least one cut 24 may be angled across the gap, either angled from the outer surface 18 to the aperture 14 or angled across the two conductors 20A, 20B.

The at least one cut 24 splits the capacitance between the opposing surfaces 22A, 22B into two or more capacitors. One cut 24 splits the capacitance into two, while two cuts 24 split the capacitance into three. Additional cuts provide still further capacitances in the BLGR 10. Each capacitance results in an additional resonant frequency of the BLGR 10. The lower resonance is dictated by the total capacitance and the higher additional resonant frequenc(ies) are due to the partial capacitance induced by each additional cut 24.

The resonant frequency of BLGR 10 is dependent upon the inductance (L) and the capacitance (C). The inductance and the capacitance are directly dependent on structural parameters identified above, according to the equations below.

$$L = \frac{\mu N^2 \pi r^2}{l}, \qquad (1)$$

$$C = \varepsilon \frac{lw}{d}, \qquad (2)$$

$$\omega = \sqrt{\frac{1}{LC}} = \sqrt{\frac{d}{\varepsilon \mu \pi N^2 r^2 w}}. \qquad (3)$$

Wherein N is the number of loops, which since the body 12 is a unitary block, a single (1) loop is provided, $\varepsilon$ is the permittivity, and aluminum has a conductivity of 3.77 x 10^7 S/m. In the BLGR 10, the resonant frequency is independent of the gap length (1). In ion sensing applications, increasing the gap length (1) allows more sample space (and thus improved sensitivity) without modifying the resonant frequency. The resonant frequency is inversely proportional to the loop radius (r). A dielectric material placed in the gap 16 changes the permittivity as well as the capacitance of the BLGR 10.

The resonant frequency is further dependent upon the quality factor (Q). The quality factor (Q) relates the amount of energy loss relative to the energy stored for a resonant system. The energy is stored by the inductor and capacitor, and the energy is dissipated by the resistor of the system. The quality factor indicates the performance of a resonator in terms of bandwidth and losses. Q is expressed as the ratio of resonant frequency to 3dB bandwidth. A high Q value implies a low loss in the system. The higher the Q factor, the closer the system behaves as an ideal inductor. Q can be calculated as in equation (4), where $\omega_r$ is the resonant frequency $\omega_r = 2\pi f_r$:

$$Q = \omega_r \frac{(average\ energy\ stored)}{(energy\ loss\ per\ cycle)} \qquad (4)$$

Alternatively, Q can be calculated as the resonance curve full bandwidth $\Delta f$ signal (full width at half maximum power) relative to its maximum frequency $f_r$.

$$Q = \frac{\Delta f}{f_r} \qquad (5)$$

As detailed herein, it has been discovered that the electrical properties of this BLGR 10 are dependent upon these physical parameters, therefore, the rest of the resonator body 12 may be designed for other considerations, for example, to provide rigid mounting surfaces, or shape to accommodate other system components or the fluid sample tube 26. This makes the BLGR 10 as described herein convenient for integration of the disclosed sensor system into existing water supply lines for contamination monitoring, as well as a durable construction.

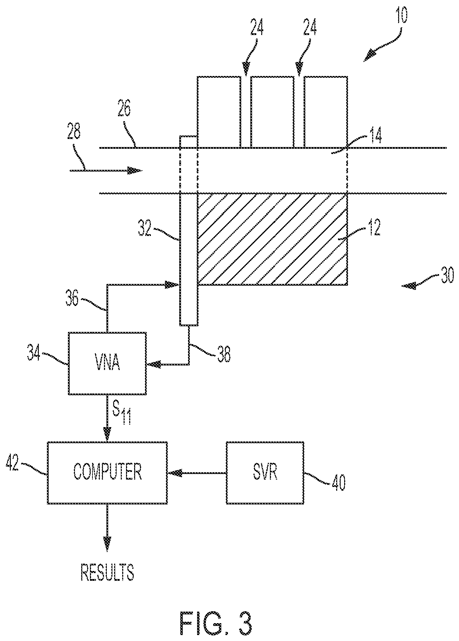

Figure 3 is a system diagram of an example of a system 30 for detection or concentration measurement of heavy metals in water. The system 30 places the BLGR 10 about a sample tube 26 which contains a flow 28 of the fluid to be tested. In an example, the fluid is water within a municipal water system. The sample tube 26 extends through the aperture 14 of the BLGR. A coupling loop 32 is exemplarily an inductive loop coupler. The BLGR 10 provides an extended surface 50 to which the coupling loop 32 is mounted, supporting the coupling loop 32 along a corresponding length of the coupling loop 32, and resulting in a sensor with improved durability. The coupling loop 32 is connected to a vector network analyzer (VNA) 34. The VNA 34 supplies an RF energy signal 36 to the coupling loop 32 which transfers such RF energy signal to the BLGR 10 by inductive coupling. The BLGR 10 reflects a portion of the RF energy signal 36 back through the coupling loop 32 to the VNA as a reflected RF energy signal 38. The VNA compares the provided RF energy signal to the reflected RF energy signal, the ratio of the two being the RF reflection spectrum (S11). Various ions in solution shift the resonant frequenc(ies) of the BLGR 10. The frequency shift depends upon charge number, concentration, and background of the solution. Solutions with different ions and concentrations of a specific ion (e.g. Pb) result in amplitude and phase variations of the S11 data. Variation can be observed over the entire range of frequency. The variation is more pronounced near the resonances of the BLGR 10.

A vector network analyzer (VNA) is an instrument that characterizes highfrequency passive and active devices and measures their effect on the amplitude and phase of swept-frequency and swept-power test signals.

A VNA generally includes operational sections: sources for stimulus, a signal-separation device, a receiver that down-converts and detects the signals, a processor for calculating results, and a graphical display connected to the processor and configured for reviewing the results. The signal source produces a stimulus for the device-under-test (e.g. the BLGR 10). The source can be used to sweep the frequency or sweep power levels of the stimulus signal. Open-loop voltage-controlled oscillators or synthesized sweepers are used to make the signal source. The signal separation block measures a portion of the incident signal using splitters or directional couplers and the incident and reflected traveling waves are separated at the input of the device under test. The measured portion of the incident signal can be used as a reference for the processor to compute the ratio-based calculations. The incident and reflected traveling waves enter the signal -detection block which may use diode detectors and tuned receivers. Conversion of RF signals to proportional DC levels is done with diode detectors. In the Receiver, local oscillators are used to translate the signal to an intermediate frequency and this signal then passes through bandpass filters. The filtered intermediate frequency signal passes through an ADC and DSP to gather magnitude and phase information before the output is transmitted, stored, and/or presented. The E8363B vector network analyzer from Agilent Technology with a frequency range of 10MHz - 40 GHz is one example of a VNA that may be used within the present disclosure.

The RF reflection spectrum (S11) is defined as the ratio of the amplitude of the reflected voltage at port one with respect to the input voltage at port one. It is a figure that quantifies the impedance discontinuity in a transmission medium. S11 is a complex quantity and the magnitude portion denoted $\Gamma$ and the phase portion is denoted with $\phi$.

$$\text{Reflection coefficient}, \text{S11} = \Gamma = \frac{\text{Vreflected}}{\text{Vincident}} = \rho\angle\phi = \frac{Z_L - Z_0}{Z_L + Z_0} \qquad (6)$$

Here, $Z_L$ is the load impedance and $Z_0$ is the transmission line impedance. When line impedance is equal to the load impedance, then all the energy will be transferred to the load and nothing will be reflected back resulting *Vreflected=0* and $\Gamma$=0. When $Z_L$ is an open or short circuit, then all the energy is reflected back and $\Gamma$=1. When $Z_L$ is not equal to $Z_0$, then some of the incident wave will be reflected back and the magnitude of the reflection coefficient will be in the range between 0 and 1.

The system 30 classifies and/or measures the concentration of ions based upon analysis of the S11 data, by application of a Support Vector Regression (SVR) algorithm trained with machine learning to detect the presence of heavy metal ions and/or to quantify the concentration of heavy metal ions in the fluid sample. Several water samples were prepared with variable trace level concentrations of Pb for machine learning training as well as for detection and quantification using the BLGR. Concentrations of 1, 3, 5, 10, and 20 ppb PbCl2 were prepared as trace level contaminants in 200 mL city water by addition of 4, 12, 20, 40, and 80 uL volumes of acidified 50 ppm Pb stock solution (e.g. PbCl2 dissolved 1% v/v 16M HNO3 in DI water), respectively. Equivalent uL volumes of a bland (0 ppb Pb) stock solution (1% v/v 16M HNO3 in DI water only) were used to prepare negative controls. Both stock solutions were prepared in DI water to minimize introduction of additional monovalent (1+) or divalent (2+) metal ions. Pb concentrations were measured with different variations of BLGRs as described herein, including but not limited to single gap, double gap, and triple gap. A total of 1500 data files of each concentration were collected with each of the resonators. The SVR algorithm is trained with test S11 data sets consisting of sensor responses to multiple known samples with different ions and ion concentrations. Magnitude and phase vectors corresponding to each sample are normalized to the interval [0,1] and concatenated into a single feature vector. The SVR was trained based on error-correcting output codes comprising three binary support vector machine classifiers with 1300 data files from each solution and tested on an additional 200 files of each solution. The magnitude and phase vectors corresponding to each sample are normalized to the interval [0,1] and concatenated into a single feature vector. These normalized vectors were used to train and test the SVR algorithm. The magnitude and phase vectors correspond to each sample.

In another example of NaCl and $MgCl_2$ ions in water, 11 different concentrations (1000 ppm - 400ppb) liquid solutions are prepared and tested with a BLGR that operates across a frequency of 10 MHz - 10 GHz, or more. In other examples, the BLGR may operate across a frequency range of 10MHz - 7 GHz or 10 MHz - 5 GHz. Still other ranges will be recognized from the present disclosure, in particular in relation to the water exchage rates as discusssed below. As will be described in further detail herein, while the BLGR may have an operating frequency range that is larger, the analysis of the S11 measurements may be limited to a frequency band, for example, a 1 GHz frequency band. Amplitude changes and frequency shifts are noticeable among different materials and concentrations. Different test materials have different radio frequencies at which they undergo excitation and the responses are identified in S11 measurement. Therefore an algorithm can be trained with appropriate datasets to identify and/or quantify the detected ions across the range of concentrations of 1000 ppm - 400ppb. A machine learning algorithm is introduced to analyze the measured S11 data. A support vector regressor (SVR) model is trained using the measured data of various salt samples. The training data is constructed by concatenating the 20,000 amplitudes and 20,000 phase values from the measured S11 data. The hyperparameters of the SVR are optimized using 10-fold cross-Validation method. In the examples herein, the SVR model can be trained with datasets to identify a plurality of ions, as with (Pb, Na, Mg, and/or K examples described), or as will be described in further detail herein, the SVR model may be trained with datasets for a single targeted ion. Based upon the trained model, the algorithm predicts the concentration(s) of ion(s) in the liquid samples. The experimental results indicate that the device can detect concentrations as low as 400 ppb with high accuracy.

In one example, a static and non-interfering heavy metal contaminant sensor makes continuous measurements for a heavy metal contaminant (Pb) in water using an AC magnetic field generated by RF resonator. In such examples, measurements down to 1 ppb Pb concentration in water may be made. The data collected by the RF S11 measurement may be processed by an algorithm trained with machine learning.

Further a block loop gap resonator (BLGR) is disclosed to improve these measurements. Examples of the resonator sensor and measurement technique are described with respect to the detection of various ions in water including, but not limited to, $Pb^{2+}$, $Na^+$, $K^{+,}$ and $Mg^{2+}$. The disclosed BLGR is sensitive to different ions and their concentration resulting in S11 amplitude and phase variations in the 10 MHz to 10GHz range. While complex mathematical models may be used to analyze the S11 data, a SVR model as described above may be used. Such analysis may result in the classification of the test ions ($Na^+$, $K^{+,}$ and $Mg^{2+}$ ) and to measure $Pb^{2+}$ concentrations in water. Information of ion concentration and classification may be more pronounced near the resonance(s) of the BLGR. In an example of a sensing system configured to detect the presence and/or concentration of a plurality of ions, an increased number of resonances in the BLGR has been found to improve detection.

In the system 30, the trained SVR 40 is stored on a computer readable medium communicatively connected to a computer 42. The computer receives the S11 data from the VNA and applies the SVR algorithm to the S11 data to produce outputs that include the identification of ions present, including for example $Na^+$, $K^+$, and $Mg^{2+}$ ions as well as $Pb^{2+}$ ions, additionally, the concentration of the $Pb^{2+}$ or other heavy metal ions is quantified.

In an example, the BLGR with more cuts 24, e.g. two cuts resulting in three resonances, was found to be the most accurate in quantifying the concentration of Pb ions in the water samples, when the results were analyzed with a general classifier SVR algorithm. Still additional cuts 24 may produce still further resonances which may help to distinguish between ions and lead to improved concentration quantification.

In another example, the BLGR may be tuned, as noted above to be sensitive to detect a particular metal ion in water. The BLGR can be tuned by the combination and size of the gap 16 and the one or more cuts 24 in the resonator body to provide a BLGR with a predetermined resonant frequency or frequencies. As will be disclosed in further detail herein, it has been $$M(H_2O)_x^{n+} + H_2O^* \rightleftharpoons M(H_2O)_{x-1}(H_2O^*)^{n+} + H_2O$$ discovered that a BLGR with a resonant frequency that matches the rate constant for water exchange with a particular metal ion is sensitive to detection of that metal ion. The water exchange rate is a unique property of metal ions in water. While there are various processes and techniques for measuring water exchange rate, including isotope dilution, nuclear magnetic resonance, or sound absorption, which may lead to variations in the determined exchange rate values, the phenomenon is represented as the following chemical equation:

(7) Margerum, D.W., G.R. Cayley, D.C. Weatherburn and G.K. Pagenkopf, 1978. Kinetics and Mechanisms of Complex Formation and Ligand Exchange, In: A.E. Martell (ed.) Coordination Chemistry, Vol. 2 ACS Monographs 174, ACS, Washington. Table 1 below provides examples of various rate constants for water exchange with metal ions. As noted, depending upon measurement technique and background conditions, there is some variation in the values of water exchange rate for various ions. Rate constant values ($k_{-w}(s^{-1})$) from three different sources are provided in Table 1, where n/r indicates where a value was not reported in the particular reference.

Table 1: Rate Constants for Water Exchange with Metal Ions

| Ion | Water Exchange Rate (Morel, Herring 1993) | Water Exchange Rate NMR measurements (Margerum 1978) | Observation Range (Margerum 1978) |
|---|---|---|---|
| $Pb^{2+}$ | $7 \times 10^9$ | n/r | n/r |
| $Hg^{2+}$ | $2 \times 10^9$ | n/r | n/r |
| $Cu^{2+}$ | $1 \times 10^9$ | $1, 5 \times 10^9$ | $(.2-5) \times 10^9$ |
| $Ca^{2+}$ | $6 \times 10^8$ | n/r | $(.5-9) \times 10^8$ |
| $Cd^{2+}$ | $3 \times 10^8$ | n/r | $(1-5) \times 10^8$ |
| $La^{3+}$ | $1 \times 10^8$ | n/r | $(.8-2) \times 10^8$ |
| $Zn^{2+}$ | $7 \times 10^7$ | n/r | $(3-50) \times 10^7$ |
| $Mn^{2+}$ | $3 \times 10^7$ | $2.3 \times 10^7$ $3.1 \times 10^7$ | $(.4-5) \times 10^7$ |
| $Fe^{2+}$ | $4 \times 10^6$ | $3.2 \times 10^6$ | $(1-5) \times 10^6$ |
| $Co^{2+}$ | $2 \times 10^6$ | $1.1 \times 10^6$ $2.2 \times 10^6$ $2.4 \times 10^6$ | $(.2-2) \times 10^6$ |
| $Mg^{2+}$ | $3 \times 10^5$ | $5.3 \times 10^5$ | $(1-5) \times 10^5$ |
| $Ni^{2+}$ | $3 \times 10^4$ | $2.7 \times 10^4$ $3.0 \times 10^4$ $3.6 \times 10^4$ | $(1-4) \times 10^4$ |
| $Fe^{3+}$ | $2 \times 10^2$ | $1.5 \times 10^2$ | $(2-200) \times 10^2$ |
| $Ga^{3+}$ | $8 \times 10^2$ | $7.6 \times 10^2$ | $(8-20) \times 10^2$ |
| $Al^{3+}$ | 1 | 16 | $(.2-16)$ |
| $Cr^{3+}$ | $5 \times 10^{-7}$ | $4.3, 5.8 \times 10^{-7}$ | $(4-6) \times 10^{-7}$ |
| $Ph^{3+}$ | $3 \times 10^{-8}$ | $3 \times 10^{-8}$ | n/r |
| $Fe^{3+}$ | $2 \times 10^2$ | $1.5 \times 10^2$ | $(2-200) \times 10^2$ |
| $FeOH^{2+}$ | $1 \times 10^5$ | n/r | n/r |

(continued)

| Ion | Water Exchange Rate (Morel, Herring 1993) | Water Exchange Rate NMR measurements (Margerum 1978) | Observation Range (Margerum 1978) |
|---|---|---|---|
| $Fe(OH)_2^+$ | $1 \times 10^7$ | n/r | n/r |
| $Fe(OH)_4^-$ | $1 \times 10^9$ | n/r | n/r |

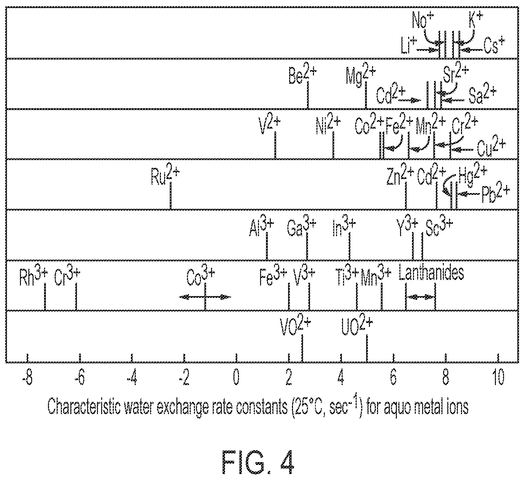

Figure 4 is a graph reproduced from Margerum, et al. which provides a further representation of example water exchange rates.

The above water exchange rates produce an ion frequency at the associated frequency (e.g. $Pb^{2+}$ $7 \times 10^9$ water exchange rate = 7 GHz ion frequency). The water exchange rate can be used in two ways to improve sensitivity of detection and/or quantification of the concentration of a target ion in a water sample.

First, as noted above, the BLGR can be tuned to have a resonant frequency that is the same as, or similar to, the resonant frequency of the target ion. As represented in the table above, differing conditions and quantification techniques result in some differences in the nominal water exchange rate for ions. Therefore further examples of BLGR as disclosed herein may include cuts to produce a BLGR with multiple resonant frequencies to either match the different nominal water exchange rates or to create a resonant frequency band from multiple resonant frequencies that cover the range of nominal water exchange rates. Using the example above for $Pb^{2+}$ ions, the BLGR is configured to produce a resonant frequency at 7GHz, and/or a combination of resonant frequencies to produce a resonant frequency band, for example of 1GHz, centered on the 7GHz frequency.

Secondly, the water exchange rates may be used to focus the training and/or implementation of the SVR model to a frequency band that is centered upon or encompasses the frequencies associated with the water exchange rate(s) for the target ion. In an example, the SVR algorithm uses a 1 GHz band centered on the frequency associated with the water exchange rate, average water exchange rate, weighted average water exchange rate, or water exchange rate observed range to focus the model training and/or focus the application of the SVR model. In an example of the use of the water exchange rate to focus the training of the SVR model to produce an ion-specific detection model, the training is focused on a frequency band including the frequency associated with the water exchange rate of the target ion. In an example of the targeted application of the SVR algorithm using the water exchange rate, the training of the SVR model is focused on a 1 GHz frequency band centered on the 7GHz frequency. During analysis, this same 1GHz frequency band of the S11 output signal from the BLGR is analyzed using the SVR model to detect or quantify the target ion in the water sample. In the example above, It will be recognized that these techniques that apply the water exchange rate may be used alone or in combination with one another.

It has further been recognized that resonators with low Q take into account a relatively large frequency range in sensing applications. Therefore, a BLGR 10 with a higher tube radius (r) or larger length (1) will result in a higher Q. Therefore, in examples, BLGR 10 sensors as disclosed herein may be tuned with a high Q to a narrow frequency range associated with particular ions to produce a highly sensitive sensor to the target ion, or the BLGR 10 sensors may be tuned with a low Q to provide sensing across a wide frequency range with the ability to make robust detection of the presence of a wide range of ions.

In the above description, certain terms have been used for brevity, clarity, and understanding. No unnecessary limitations are to be inferred therefrom beyond the requirement of the prior art because such terms are used for descriptive purposes and are intended to be broadly construed. The different systems and method steps described herein may be used alone or in combination with other systems and methods. It is to be expected that various equivalents, alternatives, and modifications are possible within the scope of the appended claims.

**Claims**

1.  A resonator (10) comprising:

    a body (12), the body having a planar surface (18);
    an aperture (14) through the body, the aperture configured to receive a tube configured for a fluid to be tested;
    a gap (16) that extends into the body from the planar surface to the aperture; and
    at least one cut (24) through the body from the planar surface towards the aperture, wherein the at least one cut extends across the gap.

2.  The resonator of claim 1, wherein the body (12) is cuboid.

3. The resonator of claim 1 or 2, wherein the cut (24) is perpendicular to the gap.

4. The resonator of any of claims 1-3, wherein the at least one cut (24) extends from the planar surface (18) to the aperture (14).

5. The resonator of any of claims 1-4, wherein the at least one cut (24) extends from the planar surface (18) through the aperture (14).

6. The resonator of any of claims 1-5, wherein the body comprises at least two cuts (24).

7. The resonator of any of claims 1-6, wherein the resonator (10) is configured to produce a resonant frequency that corresponds to a water exchange rate of an ion to be detected by the resonator, and
preferably the ion to be detected is lead and the resonant frequency is 7GHz.

8. The resonator of any of claims 1-7, wherein the resonator further comprises a dielectric material in the gap (16).

9. The resonator of any of claims 1-8, wherein the resonator exhibits a resonant frequency for each gap or cut in the resonator.

10. A system for the detection of an ion in a fluid, the system comprising:

the resonator (10) according to any of claims 1-9;
a sample tube (26) extending through the aperture (14) of the resonator, the sample tube configured to receive a sample of the fluid with the ion;
a coupling loop (32) comprising a coil of wire, the coil of wire of the coupling loop coaxially aligned with the aperture (14) of the resonator, wherein the sample tube extends through the coupling loop;
a vector network analyzer (VNA) (34) connected to the coupling loop, wherein the VNA supplies an RF energy signal to the coupling loop, the RF energy signal transferred to the resonator by inductive coupling, and the VNA receives a reflected portion of the RF energy signal through the coupling loop and calculates a reflection coefficient; and
a processor (42) that receives the reflection coefficient and produces a determination if the ion is in the fluid based upon the reflection coefficient.

11. The system of claim 10, wherein the processor applies a support vector regressor (SVR) model (40) to the reflection coefficient to produce the determination if the ion is in the fluid; and
optionally, the processor further determines a concentration of the ion in the fluid by applying the SVR model to the reflection coefficient.

12. The system of claim 11, wherein the SVR model is produced by a machine learning algorithm trained on datasets of reflection coefficient measurements of fluids having known ion concentrations.

13. The system of claim 12, wherein the SVR model is specific to a target ion and the SR model is produced by the machine learning algorithm trained on datasets of reflection coefficient measurements of the fluid having known concentrations of the target ion, and
optionally wherein analyzing the measured reflection coefficients comprises analyzing the measured reflection coefficients within a passband centered on a frequency that corresponds to a water exchange rate of the target ion.

14. A method of detecting an ion in a fluid according to any of claims 1-13, the method comprising:

providing a resonator (10) about a tube (26) configured to receive a fluid to be tested;
generating a magnetic field with the resonator;
measuring reflection coefficients as a function of frequency across a frequency range;
analyzing the measured reflection coefficients; and
determining a detection of the ion in the fluid.

15. The method of claim 14, wherein the frequency range is between 10 MHz - 10 GHz, and preferably between 10 MHz-5GHz.

**Patentansprüche**

1. Resonator (10), umfassend:

   einen Körper (12), wobei der Körper eine ebene Oberfläche (18) aufweist;
   eine Öffnung (14) durch den Körper, wobei die Öffnung konfiguriert ist, um einen Schlauch zu empfangen, der für ein zu testendes Fluid konfiguriert ist;
   einen Spalt (16), der sich von der ebenen Oberfläche bis zur Öffnung in den Körper erstreckt; und
   mindestens einen Schnitt (24) durch den Körper von der ebenen Oberfläche zur Öffnung, wobei sich der mindestens eine Schnitt über den Spalt erstreckt.

2. Resonator nach Anspruch 1, wobei der Körper (12) quaderförmig ist.

3. Resonator nach Anspruch 1 oder 2, wobei der Schnitt (24) senkrecht zum Spalt verläuft.

4. Resonator nach einem der Ansprüche 1 bis 3, wobei sich der mindestens eine Schnitt (24) von der ebenen Oberfläche (18) zur Öffnung (14) erstreckt.

5. Resonator nach einem der Ansprüche 1 bis 4, wobei sich der mindestens eine Schnitt (24) von der ebenen Oberfläche (18) durch die Öffnung (14) erstreckt.

6. Resonator nach einem der Ansprüche 1 bis 5, wobei der Körper mindestens zwei Einschnitte (24) umfasst.

7. Resonator nach einem der Ansprüche 1 bis 6, wobei der Resonator (10) konfiguriert ist, um eine Resonanzfrequenz zu erzeugen, die einer Wasseraustauschrate eines durch den Resonator zu erfassenden Ions entspricht, und vorzugsweise das zu detektierende Ion Blei ist und die Resonanzfrequenz 7 Ghz beträgt.

8. Resonator nach einem der Ansprüche 1 bis 7, wobei der Resonator ferner ein dielektrisches Material in dem Spalt (16) umfasst.

9. Resonator nach einem der Ansprüche 1 bis 8, wobei der Resonator eine Resonanzfrequenz für jede Lücke oder jeden Einschnitt im Resonator aufweist.

10. System zum Nachweis eines Ions in einem Fluid, das System umfassend:

    den Resonator (10) nach einem der Ansprüche 1 bis 9;
    ein Probenrohr (26), das sich durch die Öffnung (14) des Resonators erstreckt, wobei das Probenrohr konfiguriert ist, um eine Probe des Fluids mit dem Ion zu empfangen;
    eine Kopplungsschleife (32), die eine Drahtspule umfasst, wobei die Drahtspule der Kopplungsschleife koaxial mit der Öffnung (14) des Resonators ausgerichtet ist, wobei sich das Probenrohr durch die Kopplungsschleife erstreckt;
    einen Vektornetzwerkanalysator (VNA) (34), der mit der Kopplungsschleife verbunden ist, wobei der VNA ein HF-Energiesignal an die Kopplungsschleife liefert, das HF-Energiesignal durch induktive Kopplung an den Resonator übertragen wird und der VNA einen reflektierten Abschnitt des HF-Energiesignals durch die Kopplungsschleife empfängt und einen Reflexionskoeffizienten berechnet; und
    einen Prozessor (42), der den Reflexionskoeffizienten empfängt und basierend auf dem Reflexionskoeffizienten bestimmt, ob sich das Ion in dem Fluid befindet.

11. System nach Anspruch 10, wobei der Prozessor ein Modell eines Stützvektorregressors (SVR) (40) auf den Reflexionskoeffizienten anwendet, um zu bestimmen, ob sich das Ion in dem Fluid befindet; und optional der Prozessor ferner eine Konzentration des Ions in dem Fluid bestimmt, indem er das SVR-Modell auf den Reflexionskoeffizienten anwendet.

12. System nach Anspruch 11, wobei das SVR-Modell von einem maschinellen Lernalgorithmus erzeugt wird, der auf Datensätzen von Reflexionskoeffizientenmessungen von Fluiden mit bekannten Ionenkonzentrationen trainiert wurde.

13. System nach Anspruch 12, wobei das SVR-Modell spezifisch für ein Zielion ist und das SR-Modell durch den maschinellen Lernalgorithmus erzeugt wird, der auf Datensätzen von Reflexionskoeffizientenmessungen des Fluids mit bekannten Konzentrationen des Zielionsions erzeugt wurde, und

wobei optional das Analysieren der gemessenen Reflexionskoeffizienten das Analysieren der gemessenen Reflexionskoeffizienten innerhalb eines Durchlassbereichs umfasst, der auf eine Frequenz zentriert ist, die einer Wasseraustauschrate des Zielions entspricht.

14. Verfahren zum Nachweisen eines Ions in einem Fluid nach einem der Ansprüche 1 bis 13, das Verfahren umfassend:

Bereitstellen eines Resonators (10) um ein Rohr (26), das konfiguriert ist, ein zu testendes Fluid zu empfangen;
Erzeugen eines Magnetfeldes mit dem Resonator;
Messen von Reflexionskoeffizienten als Funktion der Frequenz über einen Frequenzbereich;
Analysieren der gemessenen Reflexionskoeffizienten; und
Bestimmen eines Nachweises des Ions in dem Fluid.

15. Verfahren nach Anspruch 14, wobei der Frequenzbereich zwischen 10 MHz und 10 GHz und vorzugsweise zwischen 10 MHz und 5 GHz liegt.

**Revendications**

1. Résonateur (10) comprenant :

un corps (12), le corps ayant une surface plane (18) ;
une ouverture (14) à travers le corps, l'ouverture étant configurée pour recevoir un tube configuré pour un fluide à tester ;
un espace (16) qui s'étend dans le corps depuis la surface plane jusqu'à l'ouverture ; et
au moins une découpe (24) à travers le corps depuis la surface plane vers l'ouverture, dans lequel l'au moins une découpe s'étend à travers l'espace.

2. Résonateur selon la revendication 1, dans lequel le corps (12) est cuboïde.

3. Résonateur selon la revendication 1 ou 2, dans lequel la découpe (24) est perpendiculaire à l'écart.

4. Résonateur selon l'une quelconque des revendications 1 à 3, dans lequel au moins une découpe (24) s'étend de la surface plane (18) jusqu'à l'ouverture (14).

5. Résonateur selon l'une quelconque des revendications 1 à 4, dans lequel l'au moins une découpe (24) s'étend depuis la surface plane (18) à travers l'ouverture (14).

6. Résonateur selon l'une quelconque des revendications 1 à 5, dans lequel le corps comprend au moins deux découpes (24).

7. Résonateur selon l'une quelconque des revendications 1 à 6, dans lequel le résonateur (10) est configuré pour produire une fréquence de résonance qui correspond à un taux d'échange d'eau d'un ion à détecter par le résonateur, et
de préférence, l'ion à détecter est le plomb et la fréquence de résonance est de 7 GHz.

8. Résonateur selon l'une quelconque des revendications 1 à 7, dans lequel le résonateur comprend en outre un matériau diélectrique dans l'espace (16).

9. Résonateur selon l'une quelconque des revendications 1 à 8, dans lequel le résonateur présente une fréquence de résonance pour chaque espace ou coupure dans le résonateur.

10. Système de détection d'un ion dans un fluide, le système comprenant :

le résonateur (10) selon l'une quelconque des revendications 1 à 9 ;
un tube d'échantillon (26) s'étendant à travers l'ouverture (14) du résonateur, le tube d'échantillon étant configuré pour recevoir un échantillon du fluide avec l'ion ;

une boucle de couplage (32) comprenant une bobine de fil, la bobine de fil de la boucle de couplage étant alignée coaxialement avec l'ouverture (14) du résonateur, dans lequel le tube d'échantillon s'étend à travers la boucle de couplage ;

un analyseur de réseau vectoriel (VNA) (34) connecté à la boucle de couplage, dans lequel le VNA fournit un signal d'énergie RF à la boucle de couplage, le signal d'énergie RF étant transféré au résonateur par couplage inductif, et le VNA reçoit une partie réfléchie du signal d'énergie RF à travers la boucle de couplage et calcule un coefficient de réflexion ; et

un processeur (42) qui reçoit le coefficient de réflexion et produit une détermination si l'ion est dans le fluide en fonction du coefficient de réflexion.

11. Système selon la revendication 10, dans lequel le processeur applique un modèle de régresseur de vecteur de support (SVR) (40) au coefficient de réflexion pour déterminer si l'ion est dans le fluide ; et

éventuellement, le processeur détermine en outre une concentration de l'ion dans le fluide en appliquant le modèle SVR au coefficient de réflexion.

12. Système selon la revendication 11, dans lequel le modèle SVR est produit par un algorithme d'apprentissage automatique formé sur des ensembles de données de mesures de coefficient de réflexion de fluides ayant des concentrations d'ions connues.

13. Système selon la revendication 12, dans lequel le modèle SVR est spécifique à un ion cible et le modèle SR est produit par l'algorithme d'apprentissage automatique formé sur des ensembles de données de mesures de coefficient de réflexion du fluide ayant des concentrations connues de l'ion cible, et

éventuellement dans lequel l'analyse des coefficients de réflexion mesurés comprend l'analyse des coefficients de réflexion mesurés dans une bande passante centrée sur une fréquence qui correspond à un taux d'échange d'eau de l'ion cible.

14. Procédé de détection d'un ion dans un fluide selon l'une quelconque des revendications 1 à 13, le procédé comprenant :

la fourniture d'un résonateur (10) autour d'un tube (26) configuré pour recevoir un fluide à tester ;
la génération d'un champ magnétique avec le résonateur ;
la mesure des coefficients de réflexion en fonction de la fréquence sur une plage de fréquences ;
l'analyse des coefficients de réflexion mesurés ; et
la détermination d'une détection de l'ion dans le fluide.

15. Procédé selon la revendication 14, dans lequel la plage de fréquences est comprise entre 10 MHz et 10 GHz, et de préférence entre 10 MHz et 5 GHz.

FIG. 1B

FIG. 1A

EP 4 200 600 B1

FIG. 2A          FIG. 2B

FIG. 3

Characteristic water exchange rate constants (25°C, sec⁻¹) for aquo metal ions

FIG. 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63067691
- US 63074730

- US 5103180 A

**Non-patent literature cited in the description**

- Kinetics and Mechanisms of Complex Formation and Ligand Exchange. **MARGERUM, D.W.** ; **G.R. CAYLEY** ; **D.C. WEATHERBURN** ; **G.K. PAGEN-KOPF**. Coordination Chemistry. ACS Monographs, 1978, vol. 2, 174